# EUROPEAN PATENT SPECIFICATION

(11) **EP 3 969 107 B1**
(45) Date of publication and mention of the grant of the patent: **22.01.2025**
(21) Application number: 20742855.8
(22) Date of filing: 15.05.2020
(51) Int. Cl.: A61N 5/06

(54) **AESTHETIC TREATMENT OF THE SKIN**
ÄSTHETISCHE BEHANDLUNG DER HAUT
LE TRAITEMENT ESTHÉTIQUE DE LA PEAU

(30) Priority: 16.05.2019 IT 201900006929
(43) Date of publication of application: 23.03.2022
(73) Proprietor: Giuliani S.p.A., 20129 Milano (IT)
(72) Inventor: GIULIANI, Giammaria, 6926 Montagnola (CH); RINALDI, Fabio, 20129 Milano (IT); MARZANI, Barbara, 27020 Carbonara Al Ticino (IT); PINTO, Daniela, 20151 Milano (IT)
(74) Representative: Cattaneo, Elisabetta
(86) International application number: PCT/IB2020/054633
(87) International publication number: WO 2020/230101

(56) References cited:
- WO-A1-2015/149177
- WO-A1-2019/014765
- WO-A2-2013/040242
- US-A1- 2013 116 612
- US-B2- 9 144 631
- URDIALES-GÁLVEZ FERNANDO ET AL: "Concomitant Use of Hyaluronic Acid and Laser in Facial Rejuvenation", AESTHETIC PLASTIC SURGERY, SPRINGER VERLAG, NEW YORK, NY, US, vol. 43, no. 4, 9 May 2019 (2019-05-09), pages 1061 - 1070, XP036986785, ISSN: 0364-216X, [retrieved on 20190509], DOI: 10.1007/S00266-019-01393-7

## Description

### Field of the invention

The present invention relates to an aesthetic treatment of the skin.

Specifically, the present invention relates to a method for preventing and treating the typical marks of skin aging and for exerting a bio-revitalizing effect on the skin.

### Background

The skin, like all the tissues of the human organism is subject to changes and to a progressive functional and aesthetic deterioration due to the natural aging process.

Skin aging originates from a combination of genetic factors and external environmental factors. The chronological aging of the skin is largely attributable to a physiological reduction in the formation of collagen, a protein that represents about 30% by weight of the body's proteins and performs a structuring and filling action.

This effect is mainly due to the reduction in the number and functional capacity of fibroblasts and their reduced metabolic capacity.

The action of these factors is added to the action of external factors such as the exposure to solar radiation, heat or harsh temperatures which contribute to accelerating the aging of the skin, in particular of the face, being it more exposed than other areas of the body to the action of external agents, causing the formation of wrinkles, thinning, loss of elasticity and the formation of depressions.

The thinning of the epidermis and the loss of elasticity further contribute to accelerating the typical marks of skin aging.

In the cosmetic field, preparations containing substances with a moisturising, emollient and mild regenerating action are used to remedy the marks of skin aging.

Instead, in the field of aesthetic dermatology, some substances with a filling action called fillers are used which, once applied by subcutaneous injection, increase the volume of the treated areas and fill the skin depressions.

The dermo-cosmetic fillers mainly perform a mechanical action of filling the tissues, causing the stretching and relaxation of the skin which results in a reduction in the depth of epidermal corrugations.

The dermo-cosmetic fillers can be divided into biodegradable fillers, the most widespread category and non-biodegradable fillers.

The non-biodegradable fillers, also called permanent fillers, are not subject to hydrolysis or phagocytosis and therefore cannot be absorbed. These fillers exert a prolonged supporting action. However, their use presents greater risks of allergic reactions and rejection by the body.

Most common permanent fillers consist of liquid silicones, acrylic hydrogels crosslinked generally with hyaluronic acid, polymethylmethacrylate microspheres dispersed in collagen and polyacrylamide gel.

Unlike permanent fillers, biodegradable fillers have a limited action over time and therefore their application needs to be renewed to keep the filling effect unchanged.

The biodegradable fillers are generally classified as intermediate and long lasting fillers. The former are made of polymers subject to enzymatic hydrolysis and are therefore reabsorbed into the tissues a few months after implantation. These fillers are made up of biodegradable polymers generally present in nature and provided with high biocompatibility and affinity with water such as for example the hyaluronic acid. However, these fillers progressively lose their initial ability to fill and stretch the tissues.

Longer-lasting fillers are made up of cross-linked polymers obtained by treating biodegradable polymers with cross-linking agents so as to form covalent bonds that make the polymer matrix less subject to enzymatic degradation when injected into the tissues.

The cross-linked polymers, such as for example the cross-linked hyaluronic acid, behave like substantially inert substances and perform a tissue filling function without however performing any type of revitalizing action. Use of cross-linked hyaluronic acids in skin treatments is described in publications US 2013/116612 A1 and "Concomitant Use of Hyaluronic Acid and Laser in Facial Rejuvenation" by Urdiales-Gálvez Fernando et all in Aesth Plast Surg (2019) 43: 1061-1070.

However, the use of hyaluronic acid in a cross-linked form is not free of local side effects mainly due to the chemical nature of the cross-linking agent. It has been observed that some cross-linking agents commonly used to polymerize hyaluronic acid cause the onset of local allergic reactions, once injected into the skin.

In addition to the cross-linked hyaluronic acid, long-acting fillers made of synthetic polymeric matrices are marketed. However it has been observed that the subcutaneous injection of these synthetic polymers can cause allergic reactions or skin redness.

These drawbacks have limited the use of cross-linked polymers as dermal fillers.

There is currently a need for systems to improve the aesthetic appearance of the skin affected by chronoaging and photoaging, which are alternative to those currently used.

One of the objects of the present disclosure resides in providing a cosmetic kit for preventing and reducing the aesthetic damages of the skin caused by chronological aging and/or by exposure to sunlight.

A further object consists in providing a system for the cosmetic treatment of the skin the use thereof entails a lower incidence of side effects connected to the use of commonly used cross-linked polymer-based fillers.

One of the objects of the present disclosure therefore consists in providing a cosmetic kit suitable for reducing facial wrinkles and for providing a bio-revitalizing and/or stretching action on the facial features.

### Summary of the invention

The invention is defined by the claims and provides a cosmetic, non-therapeutic method with a bio-revitalizing action for improving the appearance of the skin.

Within the technical field of the invention, the Applicant has found that by realising an in situ crosslinking of a hyaluronic acid in the free form using a laser radiation of selected wavelength, a bio-revitalizing effect free of side effects that reduces the typical marks of skin aging is obtained.

Furthermore, the inventors have observed that cross-linking a salt of hyaluronic acid and keeping it cross-linked in situ under conditions of high safety, by exposure to a laser light of a suitable frequency/wavelength, is possible.

In the kit used in the method of the invention the hyaluronic acid component, preferably in salified form, is of the non-cross-linked type. The use of a salt of non-cross-linked hyaluronic acid combined with the exposure to laser light in selected wavelengths as described herein, causes an effective subcutaneous cross-linking substantially free of secondary effects and with a high margin of safety, unlike to what may happen with the use of hyaluronic acid cross-linked with chemical cross-linking agents such as for example BODE, divinyl sulfone and methacrylate.

Advantageously, the treatment of linear hyaluronic acid with low level laser therapy, LLLT, as described herein causes a volumizing and filling effect on the skin without incurring the use of chemical substances.

The hyaluronic acid of the kit, having a greater fluidity than the cross-linked hyaluronic acid, has the advantage of allowing a greater flexibility of use since it can be injected into areas of the body where it is difficult to inject conventional cross-linked hyaluronic acid.

In view of the purposes previously referred to, the present disclosure provides a kit for use in the method of the present invention and for improving the external appearance of the skin including:
a) a hyaluronic acid preferably a physiologically acceptable salt thereof and
b) a laser device, preferably LLLT, for cross-linking by photo-induction the hyaluronic acid or its physiologically acceptable salt positioned or injected into the skin or subcutaneously by irradiation with laser light.

Typically, the kit is used in the prevention and/or cosmetic/aesthetic treatment of skin aging by cross-linking the hyaluronic acid positioned subcutaneously without resorting to the use of cross-linking agents with a reduction of the side effects and an increase in the safety of use of the kit/system of the invention.

In the field of cosmetic or aesthetic applications, the kit or system described herein is specifically indicated for use in the prevention or treatment of marks or skin imperfections typical of chrono-aging and/or the action of external agents such as ultraviolet radiation.

### Brief description of the figures

The characteristics and advantages of the present invention will become clearer from the accompanying tables in which:

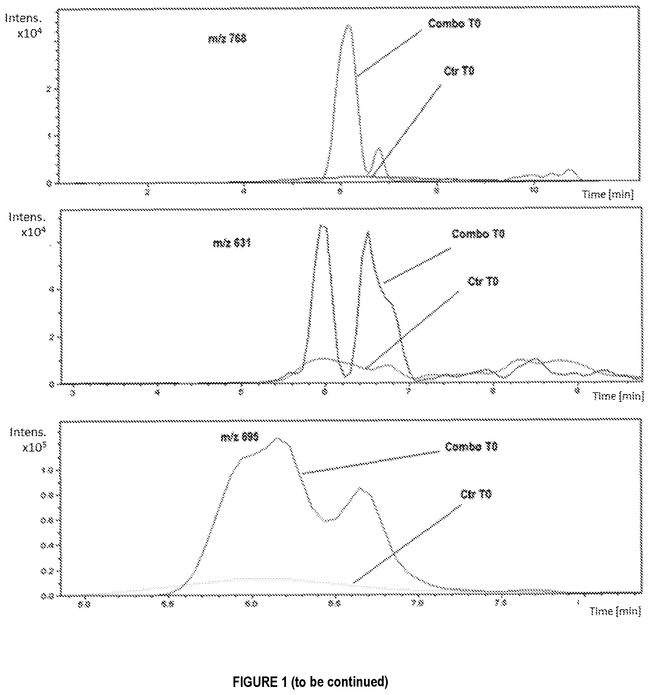

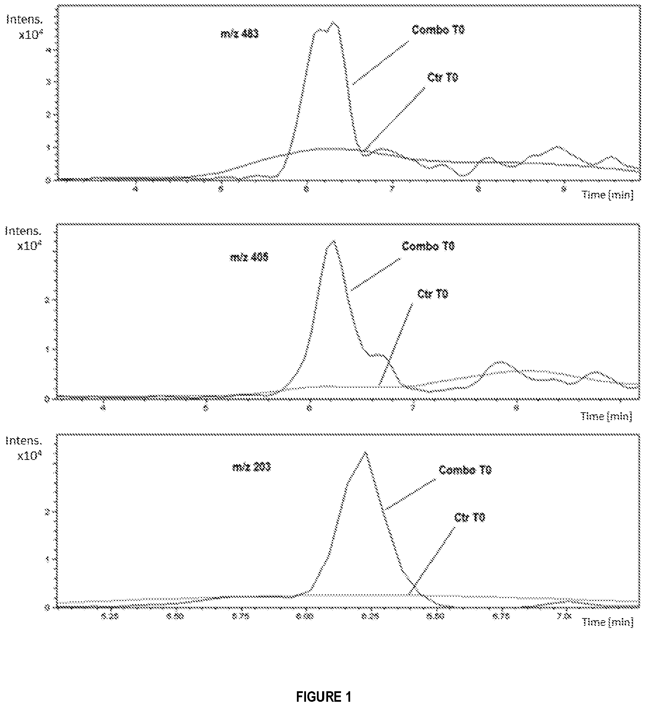

In Figure 1, the ion extraction chromatograms relative to the ions listed in Table 2 reported in Example 1 are compared;

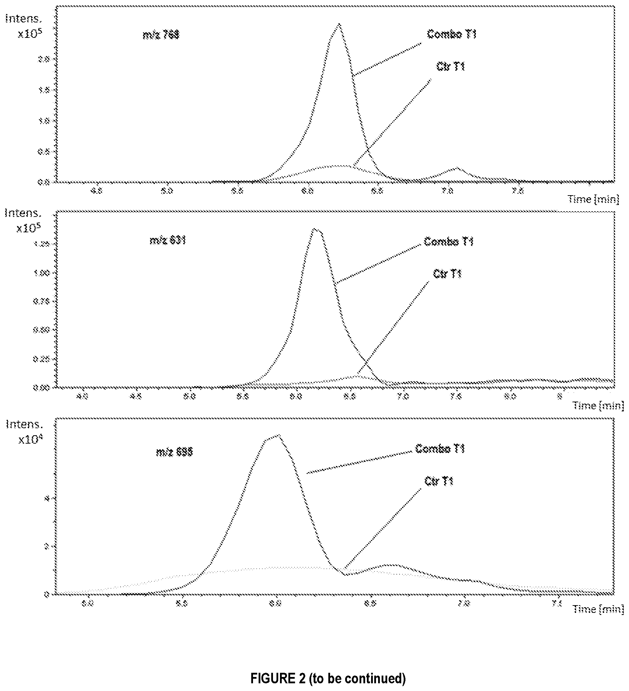

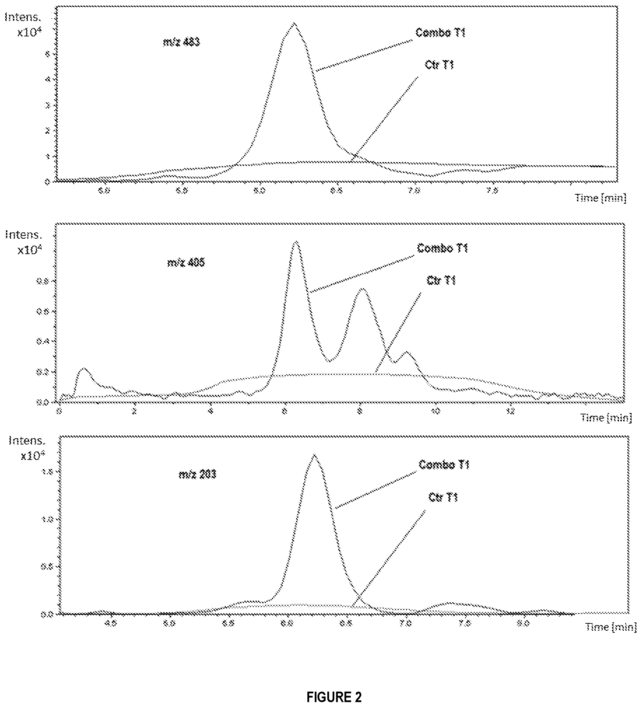

In Figure 2, the ion extraction chromatograms relative to the ions listed in Table 2 reported in Example 1 are compared:

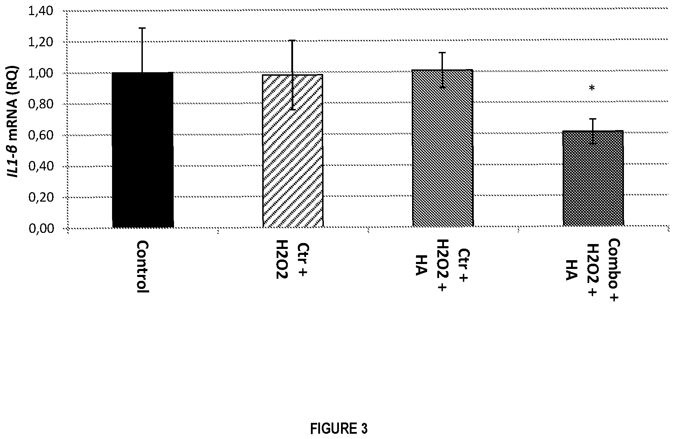

   Figure 3 illustrates bar graphs Fig. 3 relative to the gene expression of interleukin 1 beta (IL-1β) by human keratinocyte cells NCTC2544 determined in RT-PCR, as reported in Example 2. The cells were treated with: 2.5% RPMI medium alone (control); 2.5% of RPMI alone and 1mM hydrogen peroxide (Ctr + H2O2); LLLT laser (3 combined wavelengths, 450/650 and 1064nm) and 1mM hydrogen peroxide (Combo + H2O2); LLLT laser (3 combined wavelengths, 450/650 and 1064nm), hyaluronic acid and 1mM hydrogen peroxide (Combo + H2O2 + HA). The analyses were performed after incubation at 37°C for 24 hours, with a CO2 percentage of 5%. The data are the mean ± SD of three separate experiments performed in triplicate. The statistical differences between the mean values were determined with Tukey's HSD test. The asterisk indicates a significant difference (P <0.05) compared to the control.

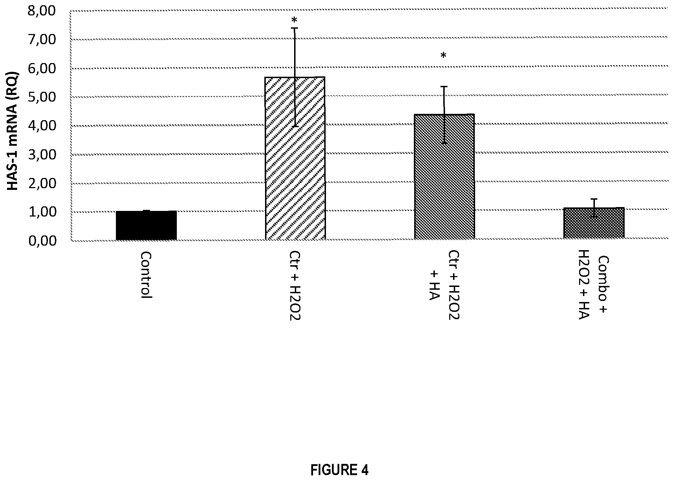

   Figure 4 illustrates bar graphs relative to the gene expression of hyaluronan synthetase 1 (HAS-1) by the murine fibroblast cells BALB3T3 determined in RT-PCR, as reported in Example 2. The cells were treated with: 2.5% DMEM medium alone (control); 2.5% of DMEM medium alone and 1mM hydrogen peroxide (Ctr + H2O2); LLLT laser (3 combined wavelengths, 450/650 and 1064nm) and 1mM hydrogen peroxide (Combo + H2O2); LLLT laser (3 combined wavelengths, 450/650 and 1064nm), hyaluronic acid and 1mM hydrogen peroxide (Combo + H2O2 + HA). The analyses were performed after incubation at 37°C for 24 hours, with a percentage of CO2 lower than 5%. The data are the mean ± SD of three separate experiments performed in triplicate. The statistical differences between the mean values were determined with Tukey's HSD test. The asterisk indicates a significant difference (P <0.05) compared to the control.

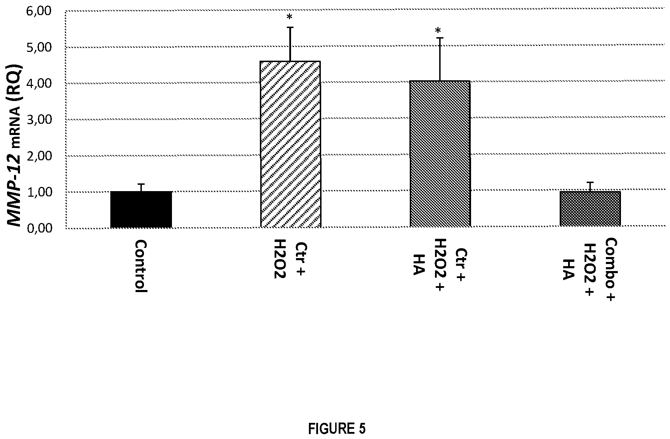

   Figure 5 illustrates bar graphs relative to the gene expression of metalloproteinase 12 (MMP-12) by murine cells of BALB3T3 fibroblasts determined in RT-PCR, as reported in Example 2. The cells were treated with: 2.5% DMEM medium alone (control); 2.5% of DMEM medium alone and 1mM hydrogen peroxide (Ctr + H2O2); LLLT laser (3 combined wavelengths, 450/650 and 1064nm) and 1mM hydrogen peroxide (Combo + H2O2); LLLT laser (3 combined wavelengths, 450/650 and 1064nm), hyaluronic acid and 1mM hydrogen peroxide (Combo + H2O2 + HA). The analyses were performed after incubation at 37°C for 24 hours, with a percentage of CO2 lower than 5%. The data are the mean ± SD of three separate experiments performed in triplicate. The statistical differences between the mean values were determined with Tukey's HSD test. The asterisk indicates a significant difference (P <0.05) compared to the control.

### Detailed description of the invention

The present invention originates from having obtained a quick cross-linking of a salt of hyaluronic acid injected into the skin by exposure to a laser light with specific wavelength.

Advantageously, the cosmetic treatment method carried out with the kit of the disclosure increases the degradation time of the salt of cross-linked hyaluronic acid subcutaneously, prolonging the dermocosmetic effect sought and allows a rapid subcutaneous cross-linking of the salt of hyaluronic acid without side effects.

Typically, the kit of the disclosure is indicated for use in the non-therapeutic field in preventing and/or treating skin aging caused by the passing of time and/or by the action of external agents.

The composition of the disclosure finds application in delaying skin aging and/or marks of skin aging.

It has been observed that by using the kit of the disclosure it is possible to cross-link the hyaluronic acid in situ, in the area where the aesthetic effect is desired. The in situ cross-linked hyaluronic acid, as described herein, stimulates fibroblasts and the regeneration of intracellular components by performing a bio-revitalizing action on the dermis and epidermis. This type of action is different from the typical filling action obtained by injecting cross-linked hyaluronic acid.

The hyaluronic acid present in the kit described herein is a non-sulfated glycosaminoglycan, that is a linear polysaccharide composed of monomeric units of D-glucuronic acid and D-N-acetylglucosamine.

The hyaluronic acid can be in a non-cross-linked free form or in a salified form with a physiologically acceptable salt, for example sodium, potassium hyaluronate. Hyaluronic acid is preferably in a salified free form as sodium hyaluronate.

A suitable hyaluronic acid or its salt can have medium-high molecular weight.

For example, a suitable hyaluronic acid or its salt for example of sodium, may have a medium-high molecular weight higher than 800.000 Da, for example ranging from 1.000.000 to 8.000.000, from 1.200.000 to 1.500.000 Daltons.

Typically, the molecular weights of hyaluronic acid or its salts, as described herein, are expressed as weight average molecular weight (MW), expressed in Dalton.

The average molecular weight of the hyaluronic acid described herein can be determined using standard methods, commonly used in the filler sector, for example as described by Ueno et al., 1988, Chem Pharm Bull. 36, 4971-4975; Wyatt 1993, Anal Chim Acta 272: 1-40; Watt Technologies 1999 "Light scattering University Dawn Course Manual and "Dawn Eos Manual" Wyatt Technology Corp. Santa Barbara CA (USA).

The hyaluronic acid or its salt present in the kit can be in the form of a gel applicable by implantation or subcutaneous injection.

The hyaluronic acid can therefore be formulated in a form suitable to be positioned/injected into a soft tissue, for example subcutaneously, typically in the dermis, in areas where it is necessary to obtain a filling or structuring effect.

A component of the kit is a laser device provided with a laser light source suitable for cross-linking the hyaluronic acid and which is conveniently absorbed by a soft tissue, typically the skin.

A suitable laser device in the kit is of the low level laser therapy type, also referred to as LLLT. Typically, the laser device of the kit irradiates selected wavelengths suitable for cross-linking by photo-induction the hyaluronic acid in the free form or its salt, positioned subcutaneously.

Preferably the laser device of the kit emits a laser light with a wavelength ranging from 450-1064 nm, preferably a light with a wavelength selected at 450, 650, 1064nm. It has been observed that by irradiating a hyaluronic acid salt subcutaneously at the selected wavelength as described herein, a reduction in cross-linking times obtained with traditional laser systems/devices is obtained.

A cosmetic method with bio-revitalizing action is provided for improving the appearance of the skin including cross-linking by photo-induction of hyaluronic acid or its physiologically acceptable salt positioned or implanted subcutaneously wherein that cross-linking occurs by irradiation with laser light with a wavelength of 450-1064 nm, emitted by a laser device.

Furthermore, the method provided increases the resistance to enzymatic degradation by hyaluronidases or to the chemical one by free radicals present subcutaneously which obliges the user to carry out frequent applications to maintain the filler effect.

The laser equipment used can be a device that amplifies the light by producing monochromatic and coherent beams of light suitable for cross-linking the hyaluronic acid present in a soft tissue, for example the subcutaneous area, of a human body. By way of example a suitable laser equipment may include a laser light source, a percutaneous irradiation unit, an optical transmission element having one end connected to the laser light source and the other end connected to the percutaneous irradiation unit.

A suitable laser device comprises a power supply base connected via a power cable to a laser applicator handpiece in which the laser sources are housed and in which there may be a button or lever for activating the laser light emission. The handpiece may have here- a gun shape with a handle. This device allows treatments to be dispensed with different wavelengths. The selection of the wavelength can be made by operating a selector positioned on the base of the power supply.

During use, the laser source, contained in the handpiece, is directed towards the area of the body, typically the face, of the subject to be treated where hyaluronic acid or its salt has previously been applied or injected subcutaneously. The activation of the laser sources takes place by pressing with the finger on the lever or actuator button. Under these conditions the laser light is emitted and directed to the area to be treated. The laser light reaches the hyaluronic acid previously applied/injected subcutaneously or in any case into the inner layers of the skin, causing its cross-linking in situ.

The Applicant has observed that hyaluronic acid, especially in the form of a salt, injected or applied in a soft tissue, typically the subcutaneous tissue, exposed to radiation/laser light with wavelength as described above is chemically modified and cross-linked with substances, mainly with glycosidic substances present in the cutaneous or subcutaneous layer.

Cross-linking protects the β-1,4 glycosidic bonds of hyaluronic acid by slowing down the enzymatic degradation process of the polysaccharide chain. Consequently, the *cross-linked* hyaluronic acid remains in the area where it has been applied/injected for a prolonged time, resulting in a prolonged filling effect, for example for 2-4 months.

Typically, the kit can be used, both for medical and non-therapeutic applications, in the treatment of the marks of skin aging and in the skin damaged by exposure to UBA, UVB or UVC rays and in general in photoaging.

A system is provided for improving the external appearance of the skin and/or increasing the volume of the cutaneous or subcutaneous tissue, said system comprising hyaluronic acid in the form of a physiologically acceptable salt and a laser device for cross-linking by photo-induction the salt of hyaluronic acid injected into the cutaneous or subcutaneous tissue by irradiation with laser light with the selected wavelengths described herein. The finds application in the cosmetic field, for example in preventing or treating chronological aging of the skin and/or by exposure to sunlight.

A cosmetic or aesthetic treatment method of the skin is provided which includes cross-linking by photo-induction of hyaluronic acid or of one of its physiologically acceptable salt injected subcutaneously by irradiation with laser light.

The present disclosure will now be described with reference to the following examples.

### EXAMPLE 1

**Table 1**

| | | |
|---|---|---|
| | Samples: | |
| | | - Ctr T0 (control samples) |
| | | - B T0 (samples in which hyaluronic acid ≥ 1.200.000 Da has been injected) |
| | | - L T0 (samples subjected to LLLT laser (coupled 450-650nm)) |
| | | - Combo T0 (samples in which hyaluronic acid ≥1.200.000 Da has been injected + LLLT laser (coupled 450-650nm)) |
| Sample description | | - Ctr T1 (control samples) |
| | | - B T1 (samples in which hyaluronic acid ≥ 1.200.000 Da has been injected) |
| | | - L T1 (samples subjected to LLLT laser (coupled 450-650nm)) |
| | | - Combo T1 (samples in which hyaluronic acid ≥1.200.000 Da has been injected + LLLT laser (coupled 450-650nm)) |
| | | |
| | Standard: | |
| | | - Hyaluronic acid |

### AIM OF THE ANALYSIS

Comparative analysis of the composition of hyaluronic acid samples under different chemical, physical and biological conditions.

### MATERIALS AND METHODS

6.2 - S.O.P. Preparation of samples with triple extraction.
3.8 - S.O.P. - AMCO analysis with single step.
3.12 - S.O.P. - AMCOESI analysis

### OBTAINED RESULTS

Comparative analyses of the molecular profiles were conducted (see file Table1.xlsx). From them, the signals overexpressed in the Combo T0 and Combo T1 samples and absent in the Ctr T0 and Ctr T1 control samples were taken into consideration. The m/z signals taken into consideration are shown in the following Table 2:

**Table 2:**

| **m/z** |
|---|
| 768.1016084 |
| 631.577331 |
| 695.0770477 |
| 483.223441 |
| 404.986113 |
| 203.6698455 |

These signals have been analysed with the aid of the Tandem Mass Spectrometry (MS/MS) technique which allows to obtain molecular/structural information through the fragmentation of the ions and the subsequent research in the library, carried out using the similarity criterion. The National Institute for Standard and Technology (NIST) library was used for this purpose.

### CONCLUSIONS OF THE STUDY

The structural study of the m/z ratios, carried out by similarity, revealed the presence of 9 branched amino-glycosidic fragments.

They indicate the presence of cross-linking reactions in the hyaluronic acid chain, irradiated with laser light inside the tissue.

### EXPLANATORY NOTES

- The data are traced through a chain of custody operating on Blockchain technology.
- The two preparations of hyaluronic acid delivered similar results.
- In Figure 1, the ion extraction chromatograms relative to the ions listed in Table 2 were compared. The traces of the Ctr T0 and Combo T0 samples were extracted.
- In Figure 2, the ion extraction chromatograms relative to the ions listed in Table 2 were compared. The traces of the Ctr T1 and Combo T1 samples were extracted.

### EXAMPLE 2

Assessment of the anti-aging efficacy of the combined treatment HA sodium salt and LLLT of the invention.

Hyaluronic acid sodium salt (referred to as HA hereinafter) injected subcutaneously was treated with LLLT (450-650-1064nm: combined treatment HA+laser). After the subcutaneous injection of linear HA, the affected area was exposed to a laser light beam by means of a 3-wavelength LLLT device. This treatment results in a reorganisation of the linear HA into a self-aggregated natural cross-linked system. The intra- and inter-chain interactions through glycosidic bonds are responsible for the formation of a temporary network structure capable of incorporating the free-circulation HA as well.

In the present experimental study, the anti-aging efficacy of the combined treatment with HA and LLLT by qRT-PCR was assessed, examining the gene expression profiles associated with skin aging on human keratinocytes NCTC 2544 and murine fibroblasts BALB3T3.

The aging process of the skin is characterized by a chronic progressive increase in the pro-inflammatory response of the cells. "Inflamm-aging" is the term coined in 2000 to describe this process and while the causes are not fully understood, the involvement of a dysregulation of the inflammatory network of cytokines has been reported.

Aging causes excessive oxidative stress and DNA damage which in turn trigger the stimulation of NF-κB and the inflammatory cascade mediated by IL-1β.

Although the injection of medium-sized HA alone did not reduce the expression of the IL-1β gene in human keratinocytes NCTC2544, compared to the negative control, as shown in Figure 3, the combined use of the LLLT laser (3 combined wavelengths, 450/650 and 1064nm) resulted in a significant reduction (p <0.05) of the IL-1β gene (Figure 3). These results confirm the usefulness of LLLT for reducing inflammation in many skin conditions, including skin aging. The results highlight a role of the photo-cross-linked HA in reducing the inflammatory cascade caused by the aging process.

The aging process has also been reported to increase the expression of hyaluronan synthetase 1 and 2. The gene expression of HAS-1 was significantly decreased (p <0.05) after the combined treatment of HA and LLLT devices, as shown in Figure 4. The increase in the level of HAS genes means a greater availability of HA, also in the photo-cross-linked form and the improvement of the skin rejuvenation process. Other proteases closely involved during the aging process of the skin are metalloproteinases. They can enzymatically accelerate the aging process from decades to hours (38). The MMPs produced by different types of skin cells have different functions and roles during aging. In particular, the MPM-12 secreted by macrophages and fibroblasts is responsible for elastin degradation. More recently, the role of MMP-12 has also been reported with regard to collagen I and III. The exposure of BALB3T3 fibroblasts to the combination of 3 wavelengths of the LLLT device after the injection of HA resulted in a significant reduction (p <0.05) of MMP-12 compared to the sample injected with HA alone and negative control, as shown in Figure 5.

### Experimental Design

Test system: NCTC2544 human keratinocytes and BALB3T3 murine fibroblasts
1) The NCTC 2544 human keratinocyte cell line was obtained from the National Institute for Cancer Research in Genoa, Italy. The cells were cultured in RPMI medium supplemented with 10% fetal bovine serum (FBS), 2 mM of L-glutamine, 1% of penicillin (10,000 U/mL) / streptomycin (10,000 µg / mL) and kept in 25 cm2 flasks of culture at 37°C, 5% CO2. Every two days the confluent cultures were split 1: 3-1: 6, after washing with phosphate buffered saline (PBS) (without Ca2 + and Mg2 +), using trypsin / EDTA and seeded with a density of 1 × 106 cells per well in 12 plates for qRT-PCR.
2) The immortalised line of murine embryonic fibroblasts BALB/c3T3, Clone A31 (ATCC, Manassas, VA, USA) was obtained from the National Cancer Research Institute (Genoa, Italy).

The cells were kept in culture in sterile 25cm3 flasks and incubated at 37°C in a 5% CO2 humid atmosphere. DMEM (Dulbecco's Modified Eagle's Medium, ATCC, Manassas, VA, USA) was used as a culture medium, to which 10% fetal bovine serum (FCS), 1% non-essential amino acids (NEAA), 1% of a mixture of penicillin and streptomycin (Pen-Strep Mix) were added. The latter reagents were all bought from Lonza Inc. (Barcelona, Spain).

During the culture phase, the 1:3 split was carried out every 2 days, upon reaching 80% of confluence, by washing with PBS 1X (Lonza, Barcelona, Spain) and detaching the cells with a trypsin-EDTA solution (Lonza, Barcelona, Spain) at 37°C for 2 minutes.

Upon reaching 80% of confluence the cell lines were seeded in 12-unit wells at a density of 1×106 cells per well.

Upon reaching 80% of confluence, they were incubated for 2hrs (BALB3T3) and 30min (NCTC2544) with 1mM H2O2.

At the end of the incubation the cells were washed with PBS and incubated with HA 200ug/mL and immediately irradiated with laser at the three wavelengths (450, 650 and 1064nm) simultaneously for about 1 minute.

After further 24hrs the RNA was extracted and qRT-PCR performed.

The RNA for qRT-PCR analysis was extracted 24 hours after treatment.

By means of the Tri Reagent (Sigma Aldrich) described by Chomczynski and Mackey (Chomczynski P, Mackey K. Modification of the TRI reagent procedure for isolation of RNA from polysaccharide- and proteoglycan-rich sources. Biotechniques 1995;19:942-5.) and the cDNA was then synthesised from 2µg of RNA template in a 20µl reaction volume, using the PrimeScript RT-PCR kit (Takara, Japan). The cDNA was amplified and detected by the Stratagene Mx3000P system in Real Time PCR (Agilent Technologies Italia S.p.A., Milan, Italy). The amplification of cDNA from NCTC2544 cells was carried out using the following Taqman probes: Hs01555410_m1 (interleukin-1 beta, IL-113), Hs999999 m1 (human glyceraldehyde-3-phosphate dehydrogenase, GAPDH). GAPDH was used as a housekeeping gene. The amplification of cDNA from BALB3T3 cells was carried out using the following Taqman probes: Mm03048195_m1 (hyaluronan synthetase, HAS1), Mm00500554_m1 (metalloproteinase-12, MMP-12), Mm00466519ml (beta-actin, β-actin, housekeeping).

The PCR amplifications were carried out in a total volume of 20 µl. The reaction mixture contained 10 µl of 2X Premix Ex Taq (Takara, Japan), 1 µl of 20 × TaqMan gene expression assay, 0,4 µl of RoX Reference Dye II (Takara, Giappone), 4,6 µl of water and 4 µl of DNA. The PCR conditions were as follows: 95 °C for 30 seconds followed by 40 cycles of 95 °C for 5 seconds, 60 °C for 20 seconds. The PCR reactions were carried out in duplicate using an MX3000p PCR machine (Stratagene, La Jolla, CA). The Δ of the cycle threshold (Vigetti D, Viola M, Karousou E, Rizzi M, Moretto P, Genasetti A, et al.. Hyaluronan-CD44-ERK1/2 regulate human aortic smooth muscle cell motility during aging. J Biol Chem. J Biol Chem 2008; 283: 4448-58) was used to calculate the relative abundance in the expression of each gene.

## Claims

1. A cosmetic, non-therapeutic method with a bio-revitalizing action for improving the appearance of the skin comprising the cross-linking by photo-induction of hyaluronic acid or a physiologically acceptable salt thereof positioned or implanted subcutaneously **characterized in that** cross-linking is carried out by irradiation with laser light with a combination of three wavelengths of 450, 650 and 1064 nm, emitted by a laser device of low level laser therapy, LLLT, type.

2. The cosmetic method according to claim 1 wherein the hyaluronic acid is in the form of a gel.

3. The method according to claim 1 or 2 wherein the physiologically acceptable salt of the hyaluronic acid is in the form of sodium or potassium salt.

4. The method according to anyone of claims 1-3 wherein the hyaluronic acid or its physiologically acceptable salt has a molecular weight higher than 1.000.000 Daltons.

5. The method according to claim 4, wherein the hyaluronic acid or its physiologically acceptable salt has a molecular weight ranging from 1.500.000 to 3.500.000 Daltons.

## Patentansprüche

1. Kosmetisches, nicht therapeutisches Verfahren mit biorevitalisierender Wirkung zur Verbesserung des Hautbilds, das die Vernetzung von Hyaluronsäure oder einem physiologisch unbedenklichen Salz davon, die/das subkutan positioniert oder implantiert ist, durch Photoinduktion umfasst, **dadurch gekennzeichnet, dass** die Vernetzung durch Bestrahlung mit Laserlicht mit einer Kombination aus drei Wellenlängen von 450, 650 und 1064 nm durchgeführt wird, das von einer Laservorrichtung vom Typ Niedrigenergie-Lasertherapie (LLLT, low level laser therapy) emittiert wird.

2. Kosmetisches Verfahren nach Anspruch 1, wobei die Hyaluronsäure in Form eines Gels vorliegt.

3. Verfahren nach Anspruch 1 oder 2, wobei das physiologisch unbedenkliche Salz der Hyaluronsäure in Form von Natrium- oder Kaliumsalz vorliegt.

4. Verfahren nach einem der Ansprüche 1-3, wobei die Hyaluronsäure oder ihr physiologisch unbedenkliches Salz ein Molekulargewicht von mehr als 1.000.000 Dalton aufweist.

5. Verfahren nach Anspruch 4, wobei die Hyaluronsäure oder ihr physiologisch unbedenkliches Salz ein Molekulargewicht im Bereich von 1.500.000 bis 3.500.000 Dalton aufweist.

## Revendications

1. Procédé cosmétique non thérapeutique à action bio-revitalisante pour améliorer l'aspect de la peau, comprenant la réticulation par photo-induction d'acide hyaluronique ou d'un sel physiologiquement acceptable de celui-ci positionné ou implanté par voie sous-cutanée, **caractérisé en ce que** la réticulation est réalisée par irradiation avec une lumière laser avec une combinaison de trois longueurs d'onde de 450, 650 et 1064 nm, émise par un dispositif laser de type thérapie laser de bas niveau, LLLT.

2. Procédé cosmétique selon la revendication 1, dans lequel l'acide hyaluronique est sous la forme d'un gel.

3. Procédé selon la revendication 1 ou 2, dans lequel le sel physiologiquement acceptable de l'acide hyaluronique est sous la forme de sel de sodium ou de potassium.

4. Procédé selon l'une quelconque des revendications 1 à 3, dans lequel l'acide hyaluronique ou son sel physiologiquement acceptable a un poids moléculaire supérieur à 1.000.000 Daltons.

5. Procédé selon la revendication 4, dans lequel l'acide hyaluronique ou son sel physiologiquement acceptable a un poids moléculaire allant de 1.500.000 à 3.500.000 Daltons.
